# EUROPEAN PATENT APPLICATION

(11) **EP 2 072 559 A1**
(43) Date of publication of application: **24.06.2009**
(21) Application number: 07024712.7
(22) Date of filing: 20.12.2007
(51) Int. Cl.: C08G 65/332, C07C 69/54, C04B 24/26

(54) **Defoamers**

(71) Applicant: Cognis IP Management GmbH, 40589 Düsseldorf (DE)
(72) Inventor: Andrioletti, Florence, 01700 Miribel (FR); Merlet, Stéphanie, 91100 Corbeil-Essonnes (FR); Lapere, Jeremie, FR-77100 Nanteuil les Meaux (FR); Marques, Maria Da Silva, 77000 Vaux Le Penil (FR); Lamarca, William, 77124 Cregy les Meaux (FR); Abribat, Benoit, 77310 Saint Fargeau Ponthierry (FR)
(74) Representative: Reinhardt, Jürgen

(57) **Abstract**

The present invention is related to the area of additives for the construction industry and concerns new defoamers based on unsaturated esters of fatty alcohol polyglycolethers, which optionally can by subjected to copolymerisation to form grafted polymers, a process for making said esters and their use as additives.
Suggested are fatty alcohol+EO+PO (meth)acrylates according to general formula (I),

**R²O(CH₂CH₂O)ₚ₁(CHCH₃CH₂O)_{q}(CH₂CH₂O)ₚ₂OC-CR¹=CH₂** **I**

in which R¹ stands for hydrogen or a methyl group, R² represents an alkyl or alkenyl radical having 12 to 22 carbon atoms, p1 and p2 independently stand for 0 or integers of 1 to 50, and q for an integer of from 1 to 50.

## Description

### Field of the invention

The present invention is related to the area of additives for the construction industry und concerns new defoamers based on unsaturated esters of fatty alcohol polygylcolethers, which optionally can be subjected to copolymerisation to form grafted polymers, a process for making said esters and their use as additives.

### Background of the invention

Polyolefinic acids, particularly polyacrylic acids, grafted with polyglycolethers and mono-methylpolyglycolethers (MPEG) and their use as additives for the manufacture of concrete are well known from literature. Examples for the state of the art can be found *inter alia* in FR 2776285 B1 (Chryso), EP 1260536 A1 **(BASF)** or WO 97/039037 A1 (Mbt). In this context, reference is also made to international patent application WO 06/050850 A1 (Cognis) disclosing anionic polymers useful as super-plasticizers for concrete, which are obtained by polymerisation of (meth)acrylic acid or their esters with dipropylendiglycolacrylate (DPGDA), tripropylenglycoldiacrylate (TPGDA), acrylamidomethyl propanesulfonic acid (AMPS) and/or acryl acetate (AA) and subsequent treatment of the intermediates with mixtures of short- and long-chain alkylpolyalkyleneglycols.

The products which can be found in the market, however, have in common that their ability to reduce foam during the processing of the concrete or cement compositions is poor and in particular their performance in removing occluded air bubbles leaves room for improvement.

Therefore the problem which underlies the present invention was to develop new defoamers in order to overcome the disadvantages known from the state of the art. In particular, the new products should show improved properties in reducing foam during the preparation of the cement or concrete.

### Detailed description of the invention

The present invention refers to fatty alcohol+EO+PO (meth)acrylates according to general formula **(I)**,

**R²O(CH₂CH₂O)p₁(CHCH₃CH₂O)q(CH₂CH₂O)p₂OC-CR¹=CH₂** (I)

in which R¹ stands for hydrogen or a methyl group, R² represents an alkyl or alkenyl radical having 12 to 22 carbon atoms, p1 and p2 independently stand for 0 or integers of 1 to 50, and q for an integer of from 1 to 50.

Surprisingly it has been observed that the defined balance between chain length of the fatty alcohol on one side and the distribution of the EO/PO units on the other leads to an ester having improved defoaming properties. The products according to the invention are in particular efficient in removing occluded air bubbles and foams formed during concrete processing operations, what is unusual since the structure misses a polymeric backbone. However, the invention encompasses also the observation that the solubility of the products in water can be significantly increased, in case the products are subjected to copolymerisation with other unsaturated monomers in order to obtain grafted polymers. Such products also show outstanding performance as defoamers and retardants for concrete and cement.

### Fatty alcohol+EO+PO (meth)acrylates

The methacrylates according to the present invention may be derived from all saturated or insaturated, linear or branched fatty alcohols comprising 12 to 22, and preferably 16 to 18 carbon atoms. It is also possible to tolerate small amounts of less than 5 % b.w. of longer or shorter species, especially in case technical grade fatty acid mixtures (e.g. coco or palm fatty alcohol fractions) are used. As far as the polyalkyleneether chain is concerned the sequence of ethylene and propylene oxide units may be block or random wise. In total, those fatty alcohol+EO+PO (meth)acrylates according to general formula (I) are preferred, in which
o R² stands for an alkyl group having 16 to 18 carbon atoms,
o p1 represents an integer between 1 and 5, p2 stands for 0 and q means an integer between 10 and 15 (block polymers), or
o p1 and p2 represents an average integer between 1 and 5 and q means an average integer between 10 and 15 (random polymers).

A preferred example is an adduct of 3 moles EO and 13 moles PO to cetyl stearyl alcohol, which is available in the market under the trademark Agnique® DMF 250 (Cognis).

### Manufacturing process

Another embodiment of the present invention is directed to a process for making fatty alcohol+EO+PO (meth)acrylates according to general formula (I) in that
(i) C₁₂-C₂₂ fatty alcohols are subjected to block or random alkoxylation with 1 to 100, preferably 5 to 50 and more preferably 8 to 20 moles ethylene oxide and 1 to 50, preferably 5 to 25, and more preferably 10 to 20 moles propylene oxide, and
(ii) the alkoxylates thus obtained are esterified with (meth)acrylic acid.

More precisely, in a first step suitable fatty alcohols, being saturated or unsaturated, preferably cetyl alcohol or stearyl alcohol or a technical mixture, is subjected to alkoxylation according to the standard processes known from the state of the art. The distribution of ethylene glycol (EO) and propylene glycol (PO) units in the monomer may be block wise or random. For example, it is possible to add a small quantity of EO to the fatty alcohol, followed by PO and optionally another quantity of EO in order to prepare a block polymer, or to mix the quantities of EO and PO, so that a randomised chain of alkylene oxide units is added to the alcohol. Once the alkoxylate has been prepared the intermediate is reacted with acrylic acid, methacrylic acid or their mixture. Esterification usually is conducted in the presence of 0.1 to 5, and preferably 1 to 2 % b.w. of an acidic catalyst, like e.g. toluene sulphonic acid. It is advantageous to use the (meth)acrylic acid in an excess of about 5 to about 15 mol-% in order to shift the equilibrium of the reaction towards the esterification products. Once esterification is completed (acid number below 5), non-reacted acid is distilled off from the product under high vacuum. Usually, additional purification of the ester is not necessary.

### Industrial application

The (meth)acrylates according to the present invention show high ability to defoam concrete and cement compositions. Another object of the present invention therefore concerns the use of fatty alcohol+EO+PO (meth)acrylates according to general formula **(I)** as additives for concrete and cement compositions in general and in particular as defoamers.

### Grafted polymers based on the (meth)acrylates according to the invention

As explained above the invention also encompasses the observation that the solubility of the hydrophobic (meth)acrylates can be significantly increased in case they are subjected to a radical polymerisation with other unsaturated monomers. A final object of the invention is therefore directed to the use of the meth(acrylates) according to general formula (I) as monomers in a process for making grafted polymers. In particular suitable unsaturated monomers in such copolymerisation reaction to form grafted polymers with improved defoaming properties are (meth)acrylic acid or their esters with fatty alcohols having 8 to 22, preferably 12 to 20 and more preferably 16 to 18 carbon atoms. Polymerisation can be conducted according to the state of the art. The (meth)acrylates according to the present invention and the co-monomers are transferred into a flask where the polymerisation takes place. The monomers can be used in a weight ratio (a):(b) from 1:99 to 99:1 preferably 20:80 to 80:20 and most preferably 40:60 to 60:40. Usually, the polymerisation takes place in aqueous solution at elevated temperatures of about 60 °C to about 100 °C, and preferably about 80 °C and is initiated by the addition of a conventional starter, such as, for example, ammonium persulfate. Once the polymerisation is completed, the reaction mixture is cooled down and - in case acidic functions are in the molecule - treated with an alkaline base, preferably an aqueous sodium or potassium hydroxide solution in a quantity sufficient to neutralise the acidic functions in the polymer and to prepare salts. Finally, the products are diluted by addition of water in order to adjust the desired active matter content of typically 20 to 50 % b.w., and preferably about 35 % b.w.

### Examples

### Example 1

### Preparation of a grafted polymer with a polyacrylic acid backbone

1 Mol acrylic acid and 0.3 Mol Cetylstearyl + 3EO + 5PO-methacrylate (Agnique® DFM 250, Cognis GmbH) were placed in a 250-ml-polymerisation flask at room temperature. The mixture was diluted with 58.6 ml water and set under nitrogen bubbling in order to remove all traces of oxygen. Then the mixture was heated to about 80 °C and 1.4 g starter (ammonium persulfate) was added. Since the polymerisation represents an exothermic reaction, the flask was cooled in order to maintain a reaction temperature of 80 to 90 °C. Once the polymerisation had finished, the product was cooled to room temperature and treated with aqueous sodium hydroxide solution to neutralise the acidic groups in the polymer and diluted with water to adjust a polymer content of 30 % b.w.

### Example 2

### Preparation of a grafted polymer with a polymethacrylic acid backbone

1 Mol methacrylic acid and 0.25 Mol Cetylstearyl + 3EO + 5PO-methacrylate (Agnique® DFM 250, Cognis GmbH) were placed in a 250-ml-polymerisation flask at room temperature. The mixture was diluted with 58.6 ml water and set under nitrogen bubbling in order to remove all traces of oxygen. Then the mixture was heated to about 80 °C and 1.4 g starter (ammonium persulfate) was added. Since the polymerisation represents an exothermic reaction, the flask was cooled in order to maintain a reaction temperature of 80 to 90 °C. Once the polymerisation had finished, the product was cooled to room temperature and treated with aqueous sodium hydroxide solution to neutralise the acidic groups in the polymer and diluted with water to adjust a polymer content of 30 % b.w.

## Claims

1. Fatty alcohol+EO+PO (meth)acrylates according to general formula **(I)**,
**R²O(CH₂CH₂O)ₚ₁(CHCH₃CH₂O)q(CH₂CH₂O)p₂OC-CR¹=CH₂** **(I)**
in which R¹ stands for hydrogen or a methyl group, R² represents an alkyl or alkenyl radical having 12 to 22 carbon atoms, p1 and p2 independently stand for 0 or integers of 1 to 50, and q for an integer of from 1 to 50.

2. Fatty alcohol+EO+PO (meth)acrylates according to Claim 1, **characterised in that** R² stands for an alkyl radical having 16 to 18 carbon atoms.

3. Fatty alcohol+EO+PO (meth)acrylates according to Claims 1 and/or 2, **characterised in that** p1 represents an integer between 1 and 5, p2 stands for 0 and q means an integer between 10 and 15, so that the EO and PO units form a block polymer.

4. Fatty alcohol+EO+PO (meth)acrylates according to any of the pre3ceding claims 1 to 3, **characterised in that** p1 and p2 represents an average integer between 1 and 5 and q means an average integer between 10 and 15, so that the EO and PO units form a random polymer.

5. Fatty alcohol+EO+PO (meth)acrylates according to any of the preceding Claims 1 to 4, **characterised in that** R¹ means a methyl group, R² stands for a C_{16/18} alkyl radical, p1 stands for 3, p2 stands for 0 and q represents 13.

6. Process for making fatty alcohol+EO+PO (meth)acrylates according to general formula (I) in that
(i) C₁₂-C₂₂ fatty alcohols are subjected to block or random alkoxylation with 1 to 100 moles ethylene oxide and 1 to 50 moles propylene oxide, and
(ii) the alkoxylates thus obtained are esterified with (meth)acrylic acid.

7. Use of fatty alcohol+EO+PO (meth)acrylates according to Claim 1 as additives for concrete and cement compositions.

8. Use of fatty alcohol+EO+PO (meth)acrylates according to Claim 1 as defoamers.

9. Use of fatty alcohol+EO+PO (meth)acrylates according to Claim 1 as monomers in a process for making grafted polymers..
